# EUROPEAN PATENT APPLICATION

(11) **EP 3 415 908 A1**
(43) Date of publication of application: **19.12.2018**
(21) Application number: 17750381.0
(22) Date of filing: 10.02.2017
(51) Int. Cl.: G01N 33/50, A61K 31/12, A61K 31/155, A61P 35/00, A61P 43/00, G01N 33/84, A61K 31/4375, A61K 31/616, A61K 35/644, A61K 39/395, A61K 45/00

(54) **IMMUNE FUNCTION INSPECTION METHOD, CANCER PATIENT CATEGORIZATION METHOD, CANCER TREATMENT EFFICACY PREDICTION METHOD, AGENT FOR INCREASING INTRACELLULAR CALCIUM ION CONCENTRATION, AGENT FOR INCREASING SELECTIVE FUNCTION OF EFFECTOR MEMORY (EM) AND EFFECTOR (EFF) IN TUMOR TISSUE, AND METHOD FOR MONITORING EFFICACY OF CANCER DRUG**

(30) Priority: 12.02.2016 JP 2016024363; 18.10.2016 JP 2016204284
(71) Applicant: National University Corporation Okayama University, Kita-ku Okayama-shi Okayama 700-8530 (JP)
(72) Inventor: UDONO, Heiichiro, Okayama-shi Okayama 700-8530 (JP); EIKAWA, Shingo, Okayama-shi Okayama 700-8530 (JP)
(74) Representative: Müller-Boré & Partner Patentanwälte PartG mbB
(86) International application number: PCT/JP2017/005011
(87) International publication number: WO 2017/138660

(57) **Abstract**

The present invention relates to an immune function inspection method, comprising obtaining peripheral blood from a human test subject; applying an immunostimulant; inspecting changes in intracellular calcium ion concentration in peripheral blood mononuclear cells (PBMC) or CD8T cells; determining a decrease in systemic immune function when the intracellular calcium ion concentration in PBMC or CD8T cells transiently increases after the application of the immunostimulant and then returns to the state before the stimulation; and determining normality of systemic immune function when the intracellular calcium ion concentration in PBMC or CD8T cells is in an upward trend after the application of the immunostimulant.

## Description

### Technical Field

### Cross Reference to Related Applications

This application claims priority to Japanese Patent Application No. 2016-24363, filed on February 12, 2016, and Japanese Patent Application No. 2016-204284, filed on October 18, 2016, the entire contents of which are herein incorporated by reference.

The present invention relates to an immune function inspection method, a cancer patient screening method, a cancer treatment efficacy prediction method, an intracellular calcium ion concentration increasing agent, an agent for selectively increasing the function of effector memory (EM) and effector (eff) in a tumor tissue, and a method for monitoring the efficacy of a cancer treating agent.

### Background Art

In the application of cancer immunotherapy, it is necessary to know the immunity in advance.

Previously, immune response was evaluated by a method of subjecting peripheral blood lymphocytes to a stimulating culture with antigen peptide, and measuring the cytotoxic activity of the lymphocytes that are increased after 1 to 2 weeks against cancer cells, or a method of restimulating the lymphocytes with peptide used for stimulation and measuring IFNγ production.

However, since these methods require a culture period of 1 to 2 weeks, the real-time lymphocyte function in the body of the patient cannot always be reflected. The process of a stimulating culture with antigen peptide may induce non-specific lymphocytic proliferation in many cases, and may thus result in false-positiveness.

Patent Document 1 discloses that metformin serves to potentiate the efficacy of chemotherapy drugs; however, the Examples of Patent Document 1 demonstrate that metformin itself does not have a cancer treating effect.

Non-patent Document 1 discloses the action mechanism of metformin, which is a type II diabetes treating agent.

Non-Patent Document 2 suggests that metformin has an anticancer activity.

Patent Document 2 discloses that a biguanide-based antidiabetic drug containing metformin improves the function of immune exhaustion CD8+T cells.

### Citation List

### Patent Documents

Patent Document 1: JP2013-503171A
Patent Document 2: JP2014-214093A

### Non-Patent Documents

Non-Patent Document 1: Nature 439, 682-687 (2006)
Non-Patent Document 2: J Exp Clin Med 2012; 4(3): 140-144

### Summary of Invention

### Technical Problem

A major object of the present invention is to provide a technique effective for activation and evaluation of the immune system.

### Solution to Problem

The present inventors provide an immune function inspection method, a cancer patient screening method, a cancer treatment efficacy prediction method, an intracellular calcium ion concentration increasing agent, an agent for selectively increasing the function of effector memory (EM) and effector (eff) in a tumor tissue, and a method for monitoring the efficacy of a cancer treating agent, as detailed below.

Item 1: An immune function inspection method, comprising obtaining peripheral blood from a human test subject; applying an immunostimulant; inspecting changes in intracellular calcium ion concentration in peripheral blood mononuclear cells (PBMC) or CD8T cells; determining a decrease in systemic immune function when the intracellular calcium ion concentration in PBMC or CD8T cells transiently increases after the application of the immunostimulant and then returns to the state before the stimulation; and determining normality of systemic immune function when the intracellular calcium ion concentration in PBMC or CD8T cells is in an upward trend after the application of the immunostimulant.

Item 2. A method for screening a cancer patient to be subjected to a combined treatment method using at least one member selected from the group consisting of phenformin, buformin and metformin, and one or more of other cancer treating agents, comprising selecting a cancer patient who is determined to have a decreased systemic immune function in the inspection method of Item 1 as a cancer patient to be subjected to the combined treatment method using at least one member selected from the group consisting of phenformin, buformin and metformin, and one or more of other cancer treating agents.

Item 3. The method according to Item 2, wherein the one or more of other cancer treating agents is anti-PD-1 antibody, aspirin, statin, curcumin, berberine, royal jelly, or propolis.

Item 4: A method of predicting the therapeutic effect of a combined treatment method using at least one member selected from the group consisting of phenformin, buformin and metformin and one or more of other cancer treating agents with respect to a cancer patient, the method comprising the following steps (1) and (2) :
step (1) of evaluating whether the intracellular calcium ion concentration transiently increases or continuously increases when mononuclear cells (PBMC) contained in peripheral blood obtained from a cancer patient are subjected to immune stimulation; and
step (2) of assuming that the combined treatment method using at least one member selected from the group consisting of phenformin, buformin and metformin, and one or more of other cancer treating agents is highly likely to exert a therapeutic effect when the intracellular calcium ion concentration of PMBC transiently increases upon immune stimulation and then rapidly decreases.

Item 5. A method for treating a cancer patient, comprising the following steps (1) and (2) :
step (1) of evaluating whether the intracellular calcium ion concentration transiently increases or continuously increases when mononuclear cells (PBMC) contained in peripheral blood obtained from a cancer patient are subjected to immune stimulation;
step (2) of assuming that the combined treatment method using at least one member selected from the group consisting of phenformin, buformin and metformin, and one or more of other cancer treating agents is highly likely to exert a therapeutic effect when the intracellular calcium ion concentration of PMBC transiently increases upon immune stimulation and then rapidly decreases; and
step (3) of administering at least one member selected from the group consisting of phenformin, buformin and metformin, and one or more of other cancer treating agents to a patient who is assumed to have a high possibility of ensuring the therapeutic effect in step (2).

Item 6. An agent for increasing intracellular calcium ion concentration in CD8T cells by immune stimulation, the agent comprising at least one member selected from the group consisting of phenformin, buformin and metformin.

Item 7. The agent for increasing intracellular calcium ion concentration in CD8T cells by immune stimulation according to Item 6, wherein the agent selectively increases intracellular calcium ion concentration in CD8T cells in a tumor tissue.

Item 8. An agent for selectively increasing the function of effector memory (EM) and effector (eff) in a tumor tissue, the agent comprising at least one member selected from the group consisting of phenformin, buformin and metformin.

Item 9. A cancer treating agent to be administered to a cancer patient having a cancer tissue with a glucose concentration of 0.5 to 1.5 mM, the agent comprising at least one member selected from the group consisting of phenformin, buformin and metformin.

Item 10. A method for monitoring effects of a cancer treating agent, comprising the following steps (i) to (iii):
step (i) of separating intratumor lymphocytes from a cancer tissue;
step (ii) of analyzing a glucose transporter (Glut1) expression level of the separated tumor-infiltrating lymphocytes (i); and
step (iii) of determining or evaluating whether a cancer treating agent that has already been administered to a cancer patient is effective based on the analyzed Glut1 expression level.
(as used herein, "a cancer tissue" means a cancer tissue removed from a cancer patient after administration of a cancer treating agent)

Item 11. The method for monitoring effects of a cancer treating agent according to Item 9, wherein the cancer treating agent is selected from the group consisting of cancer vaccines, immune checkpoint inhibitors, phenformin, buformin and metformin.

Item 12. The method for monitoring effects of a cancer treating agent according to Item 10 or 11, wherein the Glut1 expression level is analyzed by a flow cytometer.

Item 13. The method for monitoring effects of a cancer treating agent according to any one of Items 10 to 12, wherein the tumor-infiltrating lymphocytes are CD8T cells.

Item 14. The method for monitoring effects of a cancer treating agent according to any one of Items 10 to 13, wherein, in step 3, the glucose transporter (Glut1) expression level is analyzed after the separated tumor-infiltrating lymphocytes are cultured in a medium having a glucose concentration of 0.1 mM or more for at least two hours.

### Advantageous Effects of Invention

Cancers are localized to limited part of the body. Although a tumor tissue has a small number of immune cells, sampled peripheral blood is assumed to show a normal value or a nearly normal value because the immune cells in contact with the cancer are greatly diluted; however, it has been confirmed that, in fact, the functions of the immune cells, including antigens unrelated to cancer, in the peripheral blood of cancer patients were generally decreased. Hypofunction of immune cells is facilitated by radiation treatment, aging, genetic factors, an unbalanced diet, a lack of sleep, and the like. The immune status of the whole body may be easily evaluated by measuring the intracellular calcium concentration in peripheral blood immune cells. Further, the evaluation of the immune status of the whole body enables screening of cancer patients to be subjected to a treatment using at least one member selected from the group consisting of phenformin, buformin and metformin, or preferably a combined treatment using these agents and one or more of other cancer treating agents, such as anti-PD-1 antibody.

Further, because the intracellular calcium ion concentration in PBMC including CD8T cells upon immune stimulation is significantly low in cancer patients compared with that in healthy subjects, increasing the calcium ion concentration upon immune stimulation is the key to successful immunotherapy. Since an excessive increase in calcium ion concentration to be significantly greater than the level of healthy subjects is not likely to occur, side effects, such as inflammation, allergy, etc., due to excessive activation of the immune system will not be a problem.

Because the number of cancer cells constantly increases, it is necessary to increase the rate of killing cancer cells to be greater than the cancer cell growth so as to ensure complete cancer remission. At least one member selected from the group consisting of phenformin, buformin and metformin, and anti-PD-1 antibody are capable of increasing the number of immune cells (eff and EM) needed for complete remission of cancer, and also are capable of selectively increasing the ratio of highly-active CD8T cells having high productivity of 2 or more cytokines among the three cytokines (IL-2, TNFα, IFNγ), and instead decreasing the number of CD8T cells producing one or fewer cytokines. As a result, it is possible to significantly enhance immunity against cancer cells. Furthermore, the combination of at least one member selected from the group consisting of phenformin, buformin and metformin with anti-PD-1 antibody synergistically increases the number of immune cells.

In cancer immunotherapy, effector T-cells (eff) and effector memory T-cells (EM) are important; in contrast, central memory T-cells (CM) are not important except for the case using a cancer vaccine or the like. Thus, the present invention, which selectively increases the number and function of eff and EM, is effective for increasing the number of successful cancer immunotherapies.

Further, it has been known that there are tumor tissues having a low glucose concentration of about 1 mM; since the immune function is activated even under such a low glucose environment, the present invention is particularly suitable for the treatment of cancer patients having a cancer with such a low glucose condition.

Monitoring the efficacy of a cancer treating agent may be performed by administering a cancer treating agent to a cancer patient, removing the tumor tissue from the patient, examining the glucose transporter expression level of the tumor-infiltrating lymphocytes (in particular, tumor-infiltrating CD8T lymphocytes) separated from the removed cancer tissue, and determining or evaluating the efficacy of the cancer treating agent.

### Brief Description of Drawings

Fig. 1: Detection of intracellular calcium ion in mouse tumor-infiltrating lymphocytes.
Fig. 2: Detection of memory phenotype of antigen-specific tumor-infiltrating CD8T cells and evaluation of glucose uptake in each cell fraction. The central figure shows central memory T-cells (CM), effector memory T-cells (EM), and effector T (eff). The right figure shows that effector memory T-cells (EM) and effector T-cells (eff) are capturing 2-NBDG.
Fig. 3: Detection of intracellular calcium ion in human peripheral blood mononuclear cells.

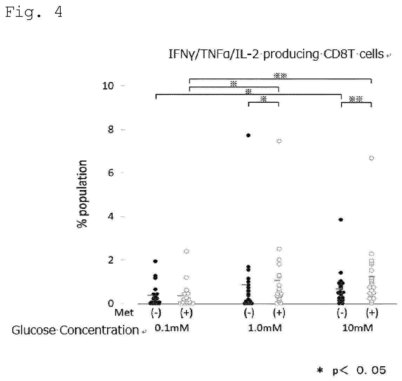

Fig. 4: Analysis of multifunctionality of human CD8T-cells under a low glucose condition. Peripheral blood lymphocytes (PBMC) w/o 10 µM metformin were cultured for 6 hours. Subsequently, after washing, the cells were cultured for 12 hours at each glucose concentration. At the final 6 hours, the cells were cultured with PMA stimulation. After the culture, intracellular cytokine was stained with an antibody, followed by FACS analysis. It was confirmed that the multifunctionality of peripheral blood CD8T cells of cancer patients were recovered by being treated with metformin in advance, even in a low glucose environment, i.e., 1.0 mM.
Fig. 5: Cancer shrinkage effects of combined use of phenformin and aspirin.
Fig. 6: An increase in expression of glucose transporter (Glut1) in tumor-infiltrating CD8T cells by administration of metformin. Expression of glucose transporter (Glut1) in tumor-infiltrating CD8T cells is increased by administration of metformin.
Fig. 7: Multifunctionality of CD8T cells expressing Glut1. The left figure reveals that many of the Glut1-positive cell groups have multifunctionality. The right figure reveals that Glut1-positive and multifunctional cell groups per tumor unit were increased in the metformin administration group. It was thus revealed that Glut1-expressing CD8T cells are regarded as a multifunctional cell group capable of simultaneously producing IFNγ, TNFα, and IL-2.
Fig. 8: Change in Glut1 expression over time.
Fig. 9: Glut1 expression versus changes in glucose concentration.
Fig. 10: An increase in calcium concentration in peripheral blood CD8T cells of cancer patients by metformin.
Fig. 11: Verification results regarding the efficacy of the combined treatment method.
Fig. 12: Verification results regarding the efficacy of the combined treatment method.
Fig. 13: Verification results regarding the efficacy of the combined treatment method.
Fig. 14: Verification results regarding the efficacy of the combined treatment method.

### Description of Embodiments

The baseline of the intracellular calcium concentration in peripheral blood immune cells (mononuclear cells (PBMC), in particular, CD8T cells) is substantially identical in healthy subjects and cancer patients. On the other hand, it is shown that, upon stimulation with an immunostimulant, such as PMA (phorbol myristic acid acetate) or ionomycin, the intracellular calcium concentration gently increases over 50 to 150 seconds in healthy human immune cells, and the necessary cytokines can thereby be released, thus maintaining a normal immune function. In contrast, in immune cells of a cancer patient, although the intracellular calcium concentration transiently increases upon stimulation with an immunostimulant, it rapidly decreases shortly thereafter. This shows that humans with decreased immune functions, including cancer patients, have a hypofunction not only in the tumor tissue, but in the immune systems of the whole body. The immune function may decrease due to the occurrence of cancer, aging, radiation treatment, overwork, lack of sleep, stress, and the like. The target test subjects of the immune function evaluation method of the present invention include all of the subjects with these conditions. The method is useful for health checking and monitoring of a cancer treating agent related to immune function. Further, since the systemic immune function is expected to decrease by cancer recurrence, the method of the present invention may also be used as a marker for cancer recurrence.

Examples of peripheral blood immune cells to be subjected to the measurement of calcium concentration include mononuclear cells (PBMC) and CD8T cells. The intracellular calcium concentration may be determined by first staining with anti-CD8 antibody, Fluo4, and FuraRed, and, after washing, performing measurement by flow cytometer. The intracellular calcium concentration may be determined by first staining with anti-CD8 antibody, Fluo4, and FuraRed, and, after washing, performing measurement by flow cytometer.

Examples of immune cells to be subjected to the evaluation of glucose transporter expression level include tumor-infiltrating lymphocytes. Tumor-infiltrating CD8T lymphocytes are preferable. Peripheral blood mononuclear cells (PBMC) have a sufficiently high blood glucose concentration unlike tumor tissues, and therefore already have a high glucose transporter expression level. Therefore, PBMC are not suitable for this inspection. The glucose transporter expression level may be determined by first staining with anti-glucose transporter antibody, anti-CD3 antibody, anti-CD8 antibody, or the like, and, after washing, performing flow cytometer analysis.

In the present invention, examples of cancer treating agents to be combined with a biguanide-based antidiabetic drug, such as phenformin, buformin, or metformin, include, but are not particularly limited to, anti-PD-1 antibody or like immune checkpoint inhibitors, aspirin, statin, curcumin, berberine, royal jelly, propolis, cancer vaccine, and the like. Among these, anti-PD-1 antibody and aspirin are more preferable. Examples of cancer treating agents to be combined also include anticancer drugs that increase the glucose transporter.

The cancer treating agent to be subjected to the monitoring method of the present invention may be a single kind of cancer treating agent or a combination of two or more cancer treating agents (combined drug). Examples of cancer treating agents to be subjected to the monitoring method are not particularly limited and may be any cancer treating agents that act on the immune system. Examples include the sole administration of a biguanide-based antidiabetic drug, such as phenformin, buformin, or metformin alone, and combined drugs of a biguanide-based antidiabetic drug such as phenformin, buformin, or metformin, and at least one member selected from the group consisting of immune checkpoint inhibitors, such as anti-PD-1 antibody, aspirin, statin, curcumin, berberine, royal jelly, propolis, and cancer vaccine. Among these, a combined drug of anti-PD-1 antibody and a biguanide-based antidiabetic drug, and a combined drug of aspirin and a biguanide-based antidiabetic drug are preferable.

In the present invention, the biguanide-based antidiabetic drug, such as phenformin, buformin, or metformin, and other cancer treating agents selected from the group consisting of immune checkpoint inhibitors, such as anti-PD-1 antibody, aspirin, statin, curcumin, berberine, royal jelly, propolis, and cancer vaccine, may be administered in the form of a mixed agent using a suitable pharmaceutical carrier, or may be administered as separate formulations in combination. The mixed agent and separate formulations may be oral agents or parenteral agents. Examples include tablets, capsules, powders, inhalations, solutions, health drinks, injections, suppositories, and the like. Adjuvants, such as antiseptics, wetting agents, emulsifiers, dispersants, stabilizers, and the like, may be added to the preparation. Further, the preparation may be administered as a suspension.

Further, solid preparations, such as tablets, pills, powdered drugs, granules, or fine granules, may be prepared, for example, by a standard method by adding carriers such as sodium bicarbonate, calcium carbonate, starch, sucrose, mannitol, or carboxymethylcellulose, and additives, such as calcium stearate, magnesium stearate, or glycerin. Further, the agent may be prepared into an enteric-coated preparation by performing enteric coating by spraying an enteric coating substance such as an organic solvent or an aqueous solution of cellulose acetate phthalate, hydroxypropylmethylcellulose phthalate, polyvinyl alcohol phthalate, styrene-maleic anhydride copolymer, methacrylic acid-methyl methacrylate copolymer, or the like. The pharmaceutically acceptable carriers may also include other general adjuvants, fragrances, and stabilizers or antiseptics, as necessary.

The cancer treating agent described above is capable of activating immune function, and is more effective for patients with decreased immune function. Therefore, when cancer chemotherapy is performed using a combination of a biguanide-based antidiabetic drug, such as phenformin, buformin, metformin and one or more of other cancer treating agents, it can be predicted that such a treatment is effective for a cancer patient with decreased immune function in which the intracellular calcium concentration is not significantly increased by immune stimulation of peripheral blood immune cells.

The side effects of phenformin, such as lactic acidosis, become problematic when the phenformin is used as an anti-diabetic agent. However, when phenformin is used for cancer treatment, the side effects are not problematic because the treatment has a dosing period and a cessation period.

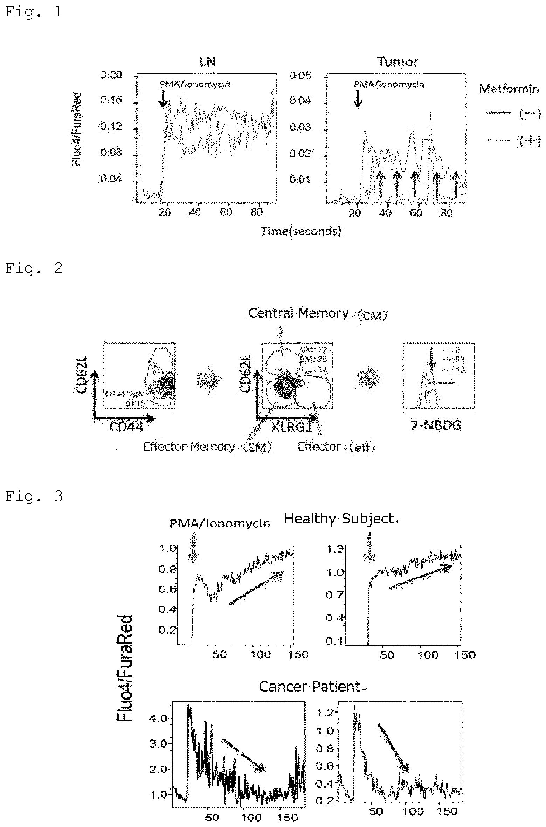

The biguanide-based antidiabetic drugs phenformin, buformin, and metformin are capable of increasing the calcium concentration in CD8T cells upon immune stimulation (Fig. 1). When immune stimulation is not performed, the intracellular calcium concentration is not changed regardless of the presence/absence of a biguanide-based antidiabetic drug. Further, the immune system activation will be no more significant than that of a healthy subject. Specifically, a biguanide-based antidiabetic drug has an effect of increasing the potential ability to increase the calcium concentration when the immune system is needed to work. The calcium concentration in the CD8T cells present in a tumor tissue (Tumor) is further lower than that of lymph nodes (LN) and hardly increases by immune stimulation. However, in the presence of the biguanide-based antidiabetic drug phenformin, buformin, or metformin, the calcium concentration in CD8T cells may be greatly increased in response to immune stimulation (Fig. 1). The dose for an adult of the biguanide-based antidiabetic drug phenformin, buformin, or metformin required for this purpose is about 100 to 150 mg for phenformin and buformin, and is about 500 to 2250 mg for metformin, per day.

Phenformin, buformin, and metformin as biguanide antidiabetic agents are capable of increasing the glucose uptake of effector (eff) and effector memory (EM) that may be localized in a tumor tissue, and does not increase the glucose uptake of central memory (CM) that is present in the secondary lymphoid tissue and that does not directly attack the cancer cells. That is, it is possible to efficiently activate T cells (eff and EM) necessary for the cancer elimination; in particular, if the treatment with a biguanide-based antidiabetic drug is not performed, it is possible to greatly increase the number and the proportion of effector (eff) that is hardly present in a tumor tissue and that has a high ability to eliminate cancer cells. The dose of the biguanide-based antidiabetic drugs phenformin and buformin for an adult required for the activation of eff and EM is about 100 to 150 mg per day, and the dose of metformin for an adult is about 500 to 2250 mg per day.

It is known that the glucose concentration is about 1 mM or less in tumor tissue (Cell 162: 1217-1228, 2015). As shown in Fig. 4, phenformin, buformin, and metformin as biguanide-based antidiabetic drugs are incapable of facilitating the cytokine production ability in CD8T cells if the glucose concentration in the tumor tissue is 0.1 mM, and becomes capable of facilitating the cytokine production ability in CD8T cells at 1 mM similar to the glucose concentration in a tumor tissue. They increase the activity of CD8T cells in a poorly nourished tumor tissue, thereby killing cancer cells. Therefore, phenformin, buformin, and metformin as biguanide-based antidiabetic drugs are useful as cancer treating agents for cancer patients with a tumor tissue having a glucose concentration of about 0.5 to 1.5 mM, preferably about 0.8 to 1.2 mM, and particularly preferably about 1 mM.

In the present invention, when the administration is performed also using one or more of other cancer treating agents, the dosages of the other cancer treating agents may be appropriately selected based on the dosages clinically used.

The monitoring of cancer treating agents may be performed by the following steps (i) to (iii):
step (i) of separating intratumor lymphocytes from a cancer tissue;
step (ii) of analyzing the glucose transporter (Glut1) expression level of tumor-infiltrating lymphocytes separated in step (i); and
step (iii) of determining or evaluating the efficacy of a cancer treating agent that has already been administered to a cancer patient based on the analyzed Glut1 expression level.

As used herein, "a cancer tissue" means a cancer tissue removed from a cancer patient after the administration of a cancer treating agent.

The monitoring of a cancer treating agent may be performed by detecting the production amounts of three kinds of cytokine (IL-2, TNFα, IFNγ); however, detection of the production amounts of these cytokines is complicated. The present inventors found that glucose transporter (Glut1) expression level is correlated with the production amounts of three kinds of cytokines, thereby enabling the monitoring of cancer treating agents by detecting a glucose transporter (Glut1) expression level. Removal of cancer tissue may be performed by surgery, or may otherwise be performed by biopsy or the like. The analysis of the glucose transporter (Glut1) expression level is preferably performed after tumor-infiltrating lymphocytes separated from a cancer patient are cultured in a medium having a glucose concentration of 0.1 mM or more for at least two hours. The Glut1 expression level is not always high in the tumor-infiltrating lymphocytes immediately after the separation from a cancer patient. By culturing them in a medium having a glucose concentration of 0.1 mM or more, it is possible to increase the Glut1 expression level and thereby eases the analysis of Glut1 expression level. The glucose concentration in a medium is about 0.1 mM or more, preferably about 1 mM or more, more preferably about 5 mM or more, further preferably about 5 to 25 mM, particularly preferably about 10 to 25 mM, and in particular, about 15 to 25 mM. The culture time is 1 hour or more, preferably 2 hours or more, more preferably 2 to 24 hours, further preferably about 3 to 12 hours, and particularly preferably about 4 to 8 hours. The culture time is set so that a high Glut1 expression level is ensured. The culture time may be short when the glucose concentration is high; when the glucose concentration is low, a long culture time is more desirable.

The period of administration of the target cancer treating agent to be monitored is not particularly limited insofar as it is at least one day. The administration period is, for example, 1 to 50 days, and preferably 2 to 30 days.

As the immune cells, lymphocytes, in particular, CD8T cells obtained from a tumor tissue are preferably used.

Examples of cancer treating agents to be subjected to the therapeutic effect evaluation include cancer vaccines, immune checkpoint inhibitors, phenformin, buformin, and metformin.

Examples of the cancer treatable by the cancer treating agent include solid cancers with a low glucose concentration, such as melanoma, head and neck cancers, esophagus cancer, gastric cancer, colon cancer, rectum cancer, liver cancer, gallbladder cancer, cholangiocarcinoma, biliary tract cancer, pancreatic cancer, lung cancer, breast cancer, ovarian cancer, cervical cancer, endometrial cancer, renal cancer, bladder cancer, prostate cancer, and the like.

Although a cancer local site has a very small number of CD8T cells, all of phenformin, buformin, and metformin as biguanide-based antidiabetic drugs and anti-PD-1 antibody are capable of increasing the number of CD8T cells in a cancer local site. Further, by combining the phenformin, buformin, and metformin as biguanide-based antidiabetic drugs with anti-PD-1 antibody, the number of CD8T cells may further be increased; moreover, the proportion of the CD8T cells capable of producing two or more cytokines among the three cytokines (IL-2, TNFα, IFNγ) may be significantly increased. The dose of the phenformin and buformin as biguanide-based antidiabetic drugs to ensure such an effect is about 100 to 150 mg for an adult per day, and the dose of metformin is about 500 to 2250 mg for an adult per day. The dose of anti-PD-1 antibody is about 2 mg/kg for an adult per day.

The present invention further provides the following inventions.

An agent for increasing the number of CD8T cells in a tumor tissue, the agent comprising anti-PD-1 antibody as an active ingredient and being administered in combination with at least one member selected from the group consisting of phenformin, buformin and metformin.

A combination of anti-PD-1 antibody and at least one member selected from the group consisting of phenformin, buformin and metformin, for increasing the number of CD8T cells in a tumor tissue.

An agent for increasing the number of CD8T cells in a tumor tissue comprising at least one member selected from the group consisting of phenformin, buformin and metformin, for use in a combination with anti-PD-1 antibody.

An agent for increasing the number of CD8T cells in a tumor tissue comprising anti-PD-1 antibody, for use in a combination with at least one member selected from the group consisting of phenformin, buformin and metformin.

An instruction for increasing the number of CD8T cells in a tumor tissue that describes a combined use of anti-PD-1 antibody with at least one member selected from the group consisting of phenformin, buformin and metformin.

### Examples

Examples are shown below to further exemplify the present invention; however, the present invention is not limited to these examples.

### Example 1

2x10⁵ MO-5 (OVA-expressing B16 melanoma cell strain) was intradermally inoculated to C57BL/6 mice; 7 days after the transplantation, free-water-drinking oral administration of 5 mg/mL metformin was started. Seven days after the administration (14 days after the transplantation), the lymph node (LN) and the tumor tissue (Tumor) were excised, and the tumor-infiltrating lymphocytes and lymphocytes of the lymph node were collected. After the cells were washed with FCS(-)RPMI, the cells were stained using antimouse CD3 antibody BV510, antimouse CD8 antibody APC-Cy7, 1 µM Fluo4, and 1 µM Fura Red for 30 minutes at 37°C. After the staining, the cells were washed with FCS(-)RPMI warmed at 37°C, followed by measurement of the background of intracellular calcium ions by a flow cytometer for 30 seconds. After the background measurement, the cells were immediately stimulated by 100 ng/ml PMA and 5 µM ionomycin, and the increase in calcium ions in the mouse CD8T cells by stimulation was measured (Fig. 1). It was confirmed that, in a tumor tissue (Tumor), although the intracellular calcium concentration was hardly increased by PMA/ionomycin stimulation in the metformin non-administration group (-), the intracellular calcium concentration was greatly increased by PMA/ionomycin stimulation in the metformin administration group (+).

The lymph node (TN) had a relatively high intracellular calcium concentration even before the PMA/ionomycin stimulation, and the intracellular calcium concentration was greatly increased by PMA/ionomycin stimulation both in the metformin administration group (+) and the metformin non-administration group (-); however, the degree of increase was greater in the metformin administration group (+).

The results of Fig. 1 revealed that metformin had an effect of increasing the intracellular calcium concentration by immune stimulation, and that the effect was significant particularly in the CD8T cells in a tumor tissue.

### Example 2

### ▪ Detection of Memory Phenotype Of Antigen-Specific Tumor-Infiltrating CD8T Cells and Evaluation Of Glucose Uptake In Each Cell Fraction

2x10⁵ MO-5 was intradermally inoculated to C57BL/6 mice; 7 days after the transplantation, free-water-drinking oral administration of 5 mg/mL metformin was started. Seven days after the administration (14 days after the transplantation), the tumor tissue was excised, and tumor-infiltrating lymphocytes were separated. After the separation, the lymphocytes were washed with 0.1% BSA/PBS, and staining was performed with antimouse CD8 antibody APC-Cy7, antimouse CD62L antibody BV421, antimouse CD44 antibody PerCP, antimouse KLRG1 antibody APC, and 400 µM 2-NBDG(2-[N-(7-nitrobenz-2-oxa-1,3-diazol-4-yl)amino]-2-deoxy-d-glucose) for 30 minutes at 4°C. After washing with 0.1% BSA/PBS, frequency and memory phenotype (CD44, CD62L, KLRG1) of antigen-specific tumor-infiltrating CD8T cells, and deoxyglucose (2-NBDG) uptake in each cell fraction were analyzed using a flow cytometer (Fig. 2). As shown in Fig. 2, it was revealed, regarding the memory phenotype of the tumor-infiltrating CD8T cells, that metformin increased the numbers and the proportions of effector (eff) and effector memory (EM) and thereby activates the glycolysis system, and that metformin however reduces the number and the proportion of central memory and thereby deactivates the glycolysis system.

### Example 3

Cryopreserved human PBMC (human peripheral blood mononuclear cells) obtained from healthy subjects and cancer patients were thawed, and washed with FCS(-)RPMI, and staining of 1x10⁶ PBMC was performed using antihuman CD8 antibody APC-Cy7, 1 µM Fluo4, and 1 µM Fura Red for 30 minutes at 37°C. After the staining, the cells were washed with FCS(-)RPMI warmed at 37°C, followed by measurement of the background of intracellular calcium ions by a flow cytometer for 30 seconds. After the background measurement, the cells were immediately stimulated by 100 ng/mL PMA and 5 µM ionomycin, and the increase in calcium ions in the human CD8T cells by stimulation was measured. Fig. 3 shows the results. Although there is no significant difference in the level before PMA/ionomycin stimulation and the increase immediately after the stimulation in PBMC of the healthy subjects and the cancer patients, there is a large difference in the subsequent progress; that is, a continuous increase was observed in the healthy subjects and a rapid decrease was observed in the cancer patients.

Human PBMC can be easily measured by drawing peripheral blood. Since the effects of metformin or the combined use of metformin and other drugs are high in the cancer patient type shown in Fig. 3, and are not high in the healthy subjects, the screening of patients may be performed by measuring the calcium concentration in human PBMC. Since the proportion of mononuclear cells in contact with cancer cells is believed to be very low in peripheral blood, this is an unexpected result.

Further, it is also possible to determine, for example, that when the calcium concentration in human PBMC is determined to be of the cancer patient type, it indicates cancer recurrence or a high possibility of cancer recurrence, and that when calcium concentration in human PBMC is determined to be of the healthy subject type, it indicates a low possibility of cancer recurrence.

### Example 4

Cryopreserved human PBMC obtained from cancer patients were thawed and cultured for 6 hours in the presence of FCS(-)RPMI 10 µM metformin. After the culture, the cells were separated and washed with FCS(-)Glc(-)RPMI. After the washing, the cells were subjected to stimulating culture for 6 hours by 50 ng/mL PMA and 2 µM ionomycin using RPMI containing 0.1, 1, 10 mM Glc in the presence of 1 µM monensin. The cells were separated and the cell-surface molecules were stained with antihuman CD8 antibody APC-Cy7, followed by a cell permeabilization treatment. The intracellular cytokines were stained with antihuman IL-2 antibody APC and antihuman TNFα antibody BV510, and antimouse IFNγ antibody FITC, followed by analysis using a flow cytometer (Fig. 4). The CD8T cells producing three cytokines (IL-2, TNFα, and IFNγ) are dependent on the glucose concentration; the number of the cells were the lowest at a glucose concentration of 0.1 mM and there is no difference depending on the presence/absence of metformin; however, at a glucose concentration of 1 mM, the number of CD8T cells producing three cytokines are significantly increased by metformin.

It is known that the glucose concentration in a poorly nourished tumor tissue is about 1 mM; therefore, the recovery of multifunctionality of the CD8T cells in such a poorly nourished tumor tissue by metformin was confirmed.

### Example 5

### Tumor Transplantation Test (phenformin, aspirin treatment)

An OVA-expressing B16 melanoma cell strain MO5 (2.5×10⁵) was intradermally inoculated to a dorsal region of C57BL/6 mice. The four types of groups: an untreated group (C), and, 10 days after the tumor cell inoculation, sole use of aspirin, sole use of phenformin (Phen), combined use of aspirin and phenformin, each consisting of 5 mice, were prepared. Phenformin is a biguanide-based drug, like metformin. From Day 10 of the tumor cell inoculation, phenformin contained in a feed in an amount of 0.5% was given. Further, the feed was replaced by a normal feed every four days. Specifically, a phenformin-containing feed was given only on Day 10 to Day 14, and Day 18 to Day 22. A normal feed free of phenformin was given from Day 14 to Day 18, and Day 22 to Day 26. This administration method is based on the fact that there have been fatal cases by everyday administration of phenformin due to side effects. Further, since the mice stopped drinking water when phenformin was given by free-water-drinking administration, phenformin was given as a feed.

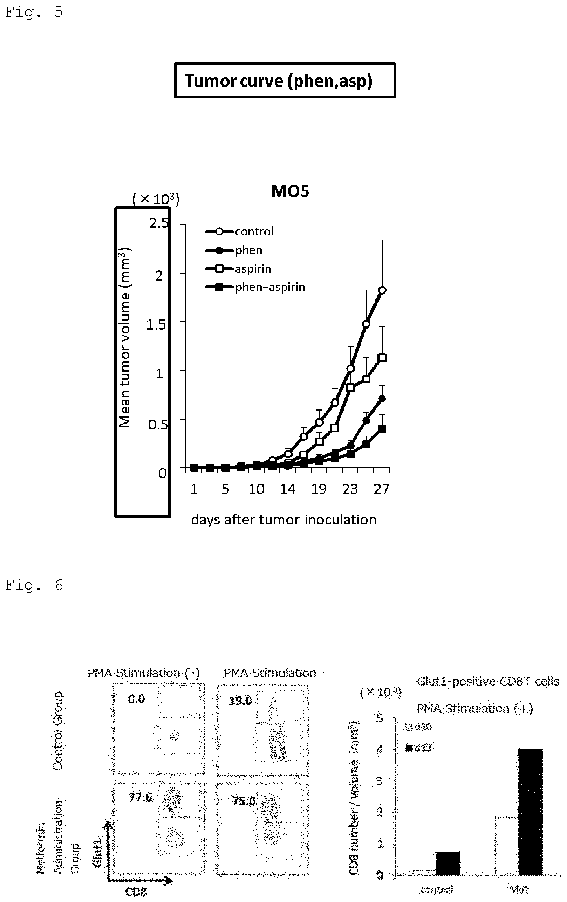

In contrast, aspirin (600 µg/mL) was given by free water drinking, and the water drinking was continuously performed from Day 10. Although strong tumor growth inhibitory effects were observed even in the sole use of phenformin, the effects were enhanced by the combined use with aspirin (Fig. 5).

### Example 6

Tumor (MethA) 2 x 10⁶ was intradermally injected to 8-week old BALB/c, and metformin (5 mg/mL) was continuously given by free-water-drinking from Day 7. Three and 6 days after the treatment (Day 10 and Day 13), the tumor-infiltrating T cells (TIL) were separated and stained with anti-CD8 antibody, and anti-glucose transporter (Glut1) antibody. Further, after stimulating culture in the presence of PMA/Ionomycin/Monencin for 6 hours, the cells were stained with anti-CD8 antibody and anti-glucose transporter (Glut1) antibody, followed by analysis using a flow cytometer. Fig. 6 shows the results. Although the Glut1 expression tends to increase by PMA stimulation, a conspicuous increase of Glut1-positive group was confirmed in the group given metformin in advance, including PMA stimulation (-).

### Example 7

The multifunctionality of the Glut1-positive group was analyzed as shown in Fig. 6. Multifunctional CD8T cells are the strongest effector T cells.

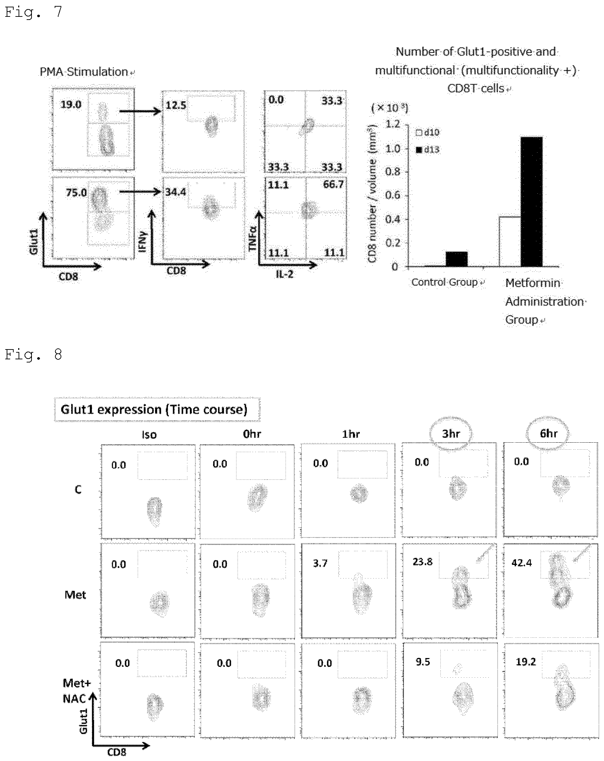

Tumor (MethA) 2 x 10⁶ was intradermally injected to 8-week old BALB/c, and metformin (5 mg/mL) was continuously given by free-water-drinking from Day 7. Three and 6 days after the treatment (Day 10 and Day 13), the tumor-infiltrating T cells (TIL) were separated. After stimulating culture in the presence of PMA/Ionomycin/Monencin for 6 hours, the cells were stained with anti-CD8 antibody, anti-glucose transporter (Glut1) antibody, and intracellular cytokines (IL-2/TNFα/IFNγ), followed by analysis using a flow cytometer. The results revealed that many Glut1-positive groups with multifunctionality were confirmed (Fig. 7). The proportion is increased by metformin administration. These facts indicate that, by confirming the Glut1 expression level in the tumor-infiltrating CD8T cells using a flow cytometer, it is possible to assume the immune status without performing a multifunctionality test. Further, by performing immunotherapy such as metformin administration, and confirming that the Glut1 expression level was increased in the CD8T cells by the therapy, it is possible to determine that the treatment had immune effects.

### Example 8

Syngeneic tumor MethA (2 x 10⁶) was intradermally injected to 8-week old BALB/c, and administration of metformin (5 mg/mL) and N-acetylcysteine (NAC, 10 mg/mL) was started from Day 7. Three days after the treatment, the tumor-infiltrating T cells (TIL) were separated, and cultured for 0, 1, 3 and 6 hours at 37 °C. Therefore, the cells were stained with CD3, CD8, and glucose transporter (Glut1) antibodies, followed by analysis using a flow cytometer (Fig. 8).

Results: Although the expression of GLUT-1 in the CD8TIL immediately after the separation from the tumor has been analyzed by staining with an antibody immediately after the separation, followed by flow cytometer analysis, the detection of Glut-1 is difficult by this method. However, it was revealed that the detection was possible by in-vitro culture for 3 to 6 hours, as shown by the arrow in Fig. 8. Specifically, a very high level GLUT-1 expression was observed in CD8TIL of the metformin-administered mice after 6-hour culture. In contrast, GLUT-1 up-regulation was not observed in the metformin non-administration group.

The above results revealed that the presence/absence of GLUT-1 up-regulation in CD8TIL by 3 to 6 in-vitro culture can serve as a monitoring method for predicting the presence/absence of metformin effects. GLUT-1 is a receptor of glucose uptake, and the up-regulation of GLUT-1 in the cell membrane indicates an acceleration of glycolysis system. Since the increase in cytoplasmic calcium ion concentration by T-cell activation is maintained by acceleration of glycolysis system, the GLUT-1 up-regulation in this case may be regarded as identical to the increase in cytoplasmic calcium ion concentration.

Further, the GLUT-1 up-regulation was significantly suppressed by administration of N-acetylcysteine (NAC), which is an antioxidant, together with metformin. Therefore, the phenomena called glycolysis system acceleration (specifically, GLUT-1 up-regulation) of CD8TIL is believed to be dependent on reactive oxygen species (ROS).

### Example 9

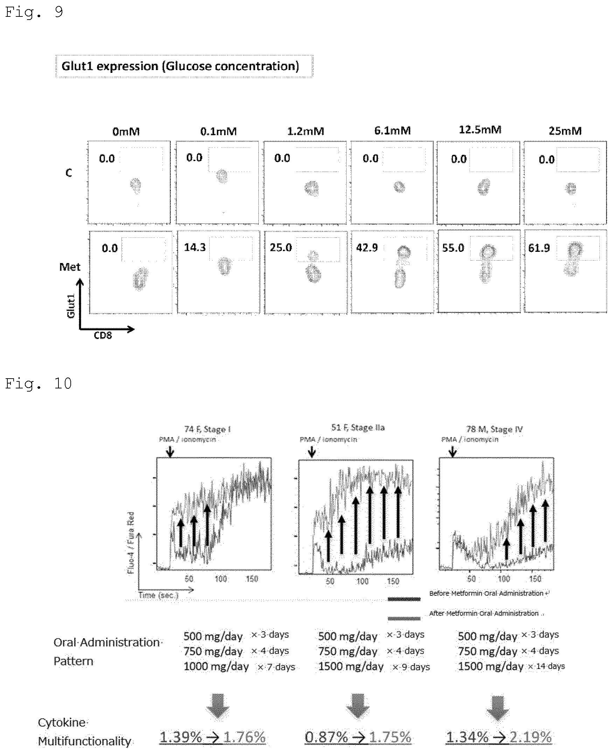

Syngeneic tumor (MethA) 2 x 10⁶ was intradermally injected to 8-week old BALB/c mice, and administration of metformin (5 mg/mL) was started from Day 7. Three days after the treatment, the tumor-infiltrating T cells (TIL) were separated. After culture in the presence of a culture solution having a glucose concentration of 0, 0.1, 1.2, 6.1, 12.5, or 25 mM at 37 °C for 0, 1, 3, 6 hours, the cells were stained with CD3, CD8, and glucose transporter (Glut1) antibodies, followed by analysis using a flow cytometer (Fig. 9).

Results: The up-regulation of GLUT-1 in the CD8TIL immediately after the separation from the tumor by 6-hour in-vitro culture was confirmed. It was revealed that this is proportional to the glucose concentration of the culture solution. In particular, the highest Glut-1 level was observed at a glucose 6.1 mM, ideally 25 mM. Glucose 25 mM is comparable to the glucose concentration in a general culture solution.

Further, Glut-1 expression was confirmed in 14.3% of CD8TIL even at a glucose concentration of 0.1 mM, which is comparable to the glucose concentration in a tumor. The glucose concentration of 0.1 mM is comparable to the glucose concentration in a tumor. Therefore, the glycolysis system in CD8TIL is assumed to accelerate also in a tumor by metformin administration, thereby attacking the tumor.

On the other hand, Glut-1 expression in CD8TIL does not occur in a complete absence of glucose (i.e., glucose concentration 0 mM). In the CD8TIL separated from the metformin-administered mice, significant up-regulation of GLUT-1 was confirmed by 6-hour in-vitro culture. It was revealed that this is proportional to the glucose concentration of the culture solution. It was revealed that the highest Glut-1 up-regulation was observed at a glucose concentration of 6.1 mM or more, 25 mM, of the culture solution.

The previous studies revealed that the effector function of tumor-infiltrating CD8T cells (CD8TIL) is dependent on the presence/absence of glycolysis system acceleration. However, it has been considerably difficult to analyze the condition of the metabolism of CD8TIL. It is thus concluded that the present method, i.e., the method of observing the Glut-1 expression level using a flow cytometer after a 6-hour culture of CD8TIL in the presence of 25 mM glucose is an excellent inspection method that is capable of significantly easy and sensitive detection of the presence or absence of glycolysis system acceleration in CD8TIL. This method is believed to be a universal method capable of monitoring the effects of not only metformin, but also various other cancer immunotherapies, including, for example, cancer vaccines, immune checkpoint inhibitors, and the like.

### Example 10

### Tumor-Infiltrating Lymphocytes (TIL) Collection and Culture Protocol

A tumor lump (diameter = 3-4 mm) was removed from a cancer patient by biopsy or the like, and mechanically pulverized for 2 minutes using Medimachine (AS ONE Corporation). The cell fluid was collected and centrifuged for 5 to 10 minutes at 1200 rpm. Thereafter, the supernatant was discarded and the cell fluid was adjusted to 5×10⁵ /mL with an RPMI culture solution (fetal bovine serum, which may be aggregated, is not added here), and the fluid was placed in a 24-well plate in an amount of 5×10⁵ /mL/well, followed by culturing for six hours. The cells were collected after 6 hours, followed by a staining step with an antibody.

### Example 11

In stage I, stage IIa, and stage IV cancer patients, changes in calcium concentration increase by PMA stimulation before and after metformin administration were measured. Human PBMC (human peripheral blood mononuclear cells) were collected before and after the metformin administration that was performed in accordance with the administration schedule shown in the figure, and subjected to stimulation with 100 ng/mL PMA and 5 µM ionomycin in the same manner as in Example 3, followed by measurement of calcium ion increase in human CD8T cells as a result of stimulation. Specifically, peripheral blood mononuclear cells (PBMC) were separated from peripheral blood by a gravity separation method, and 1x10⁶ lymphocytes were incubated for 30 minutes at 37°C in the presence of 1 µg/mL anti-human CD8 antibody and 1 µM Fluo4/FuraRed. The incubation was followed by washing with an AIM-V medium. After the washing, AIM-V was added and unstimulated (before stimulation) lymphocytes were collected by FACSCantoII for 20 minutes, thereby collecting baseline data. Thereafter, 100 ng/mL PMA and 2 µM ionomycin were added and stimulation was applied, followed by immediate collection by FACSCantoII, thereby obtaining data after stimulation. Consequently, waveforms according to the cytoplasmic calcium concentrations were detected.

Fig. 10 shows the results. In all of stage I, stage IIa, and stage IV, an increase in calcium concentration by PMA stimulation was confirmed by a culture with metformin. The results were similar to the increase in cytokine multifunctionality performed at the same time.

### Example 12

### Tumor Transplantation Test (Met, aspirin, avasimibe, and combination of 2 agents)

An OVA-expressing B16 melanoma cell strain MO5 (2.5×10⁵) was intradermally inoculated to a dorsal region of C57BL/6 mice. Seven days after tumor cell inoculation, mice were given metformin (0.5%) as a feed, aspirin (600 µg/mL) by free-water-drinking, and avasimibe (15 mg/kg) by alternate-day intraperitoneal administration.

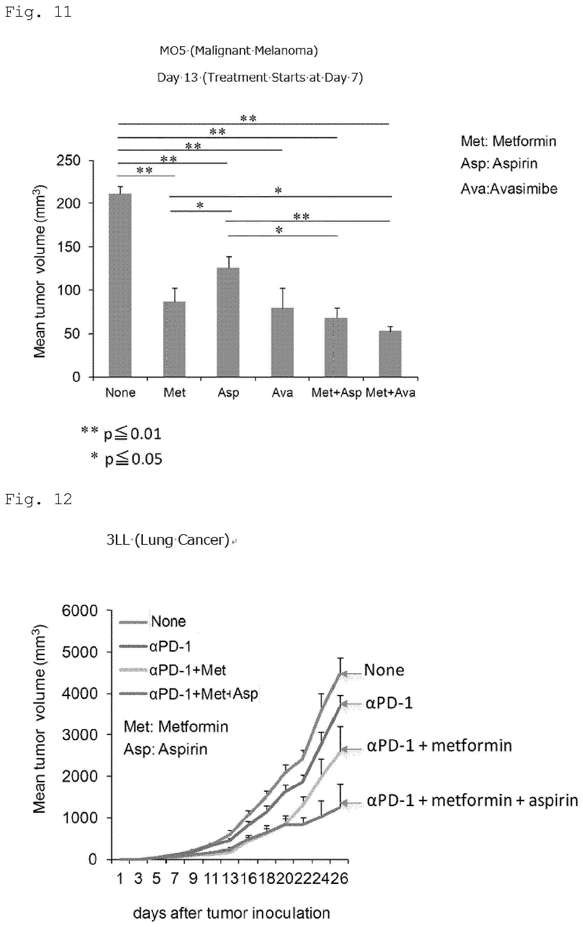

Fig. 11 shows the results. After the treatments were started, the major axis and the minor axis of the tumor on Day 6 were measured, and the volume of the tumor (mm³) was calculated. It was revealed that the tumor inhibitory effect of the combined use of metformin and aspirin, or metformin and avasimibe, was higher than that of the sole use of each agent.

### Example 13

### Tumor Transplantation Test (combination of 3 agents, anti-PD-1 antibody, Met, aspirin)

3LL cells (2.0×10⁵) were intradermally inoculated to a dorsal region of C57BL/6 mice. Five days after the inoculation of tumor cells, anti-PD-1 antibody (10 mg/kg) was administered intraperitoneally, and then further administered three more times every six days (four times in total). The treatments were performed with metformin (0.5%) as a feed, and aspirin (600 µg/mL) as free-water-drinking. After the treatments were started, the major axis and the minor axis of the tumor were measured, and the volume of the tumor (mm³) was calculated.

Fig. 12 shows the results. It was revealed that the tumor inhibitory effect of anti-PD-1 antibody and metformin was increased by the combined use with aspirin.

### Example 14

### Tumor Transplantation Test (combination of 3 agents, anti-PD-1 antibody, Met, NDGA)

3LL cells (2.0×10⁵) were intradermally inoculated to a dorsal region of C57BL/6 mice. Five days after the inoculation of tumor cells, anti-PD-1 antibody (10 mg/kg) was administered intraperitoneally, and then further administered three more times every six days (four times in total). Further, the treatments with metformin and NDGA (Nordihydroguaiaretic acid) were performed with a metformin (0.5%) feed or a mixed feed of metformin (0.5%) and NDGA (0.1%). After the treatments were started, the major axis and the minor axis of the tumor were measured, and the volume of the tumor (mm³) was calculated. Further, the survival time was observed.

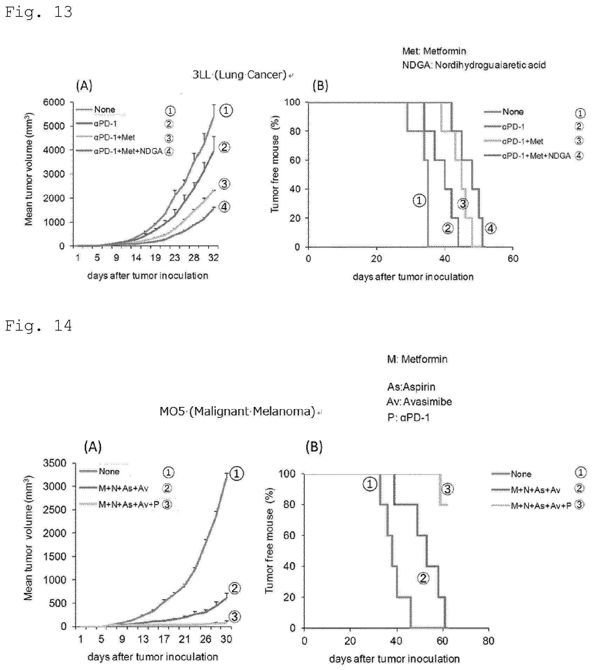

Fig. 13 shows the results. It was revealed that the tumor inhibitory effect of anti-PD-1 antibody and metformin was increased by the combined use with NDGA, thereby prolonging the survival time.

### Example 15

### Tumor Transplantation Test (combination of 4 or 5 agents)

An OVA-expressing B16 melanoma cell strain MO5 (2.5×10⁵) was intradermally inoculated to a dorsal region of C57BL/6 mice. Seven days after tumor cell inoculation, the mice were given metformin (0.5%) and NDGA (0.1%) as a mixed feed, and aspirin (600 µg/mL) by free-water-drinking. Further, anti-PD-1 antibody (10 mg/kg) was administered intraperitoneally, and then further administered three more times every six days (four times in total). Further, after the first intraperitoneal administration of avasimibe (15 mg/kg), avasimibe was further administered every two days until the final day of measurement. After the treatments were started, the major axis and the minor axis of the tumor were measured, and the volume of the tumor (mm³) was calculated. Further, the survival time was observed.

Fig. 14 shows the results. It was revealed that the combination of metformin, NDGA, aspirin and avasimibe strongly inhibited tumor growth, thereby prolonging the survival time. It was revealed that the combination of metformin, NDGA, aspirin, and avasimibe inhibited the tumor growth further strongly, thereby prolonging the survival time (complete tumor regression was confirmed in 2 mice out of 5).

## Claims

1. An immune function inspection method, comprising obtaining peripheral blood from a human test subject; applying an immunostimulant; inspecting changes in intracellular calcium ion concentration in peripheral blood mononuclear cells (PBMC) or CD8T cells; determining a decrease in systemic immune function when the intracellular calcium ion concentration in PBMC or CD8T cells transiently increases after the application of the immunostimulant and then returns to the state before the stimulation; and determining normality of systemic immune function when the intracellular calcium ion concentration in PBMC or CD8T cells is in an upward trend after the application of the immunostimulant.

2. A method for screening a cancer patient to be subjected to a combined treatment method using at least one member selected from the group consisting of phenformin, buformin and metformin, and one or more of other cancer treating agents, comprising performing the combined treatment method using at least one member selected from the group consisting of phenformin, buformin and metformin, and one or more of other cancer treating agents, with respect to a cancer patient who is determined to have a decreased systemic immune function in the inspection method of claim 1.

3. The method according to claim 2, wherein the one or more of other cancer treating agents is anti-PD-1 antibody, aspirin, statin, curcumin, berberine, royal jelly, or propolis.

4. A method of predicting the therapeutic effect of a combined treatment method using at least one member selected from the group consisting of phenformin, buformin and metformin and one or more of other cancer treating agents with respect to a cancer patient, the method comprising the following steps (1) and (2) :
step (1) of evaluating whether the intracellular calcium ion concentration transiently increases or continuously increases when mononuclear cells (PBMC) contained in peripheral blood obtained from a cancer patient are subjected to immune stimulation; and
step (2) of assuming that the combined treatment method using at least one member selected from the group consisting of phenformin, buformin and metformin, and one or more of other cancer treating agents is highly likely to exert a therapeutic effect when the intracellular calcium ion concentration of PMBC transiently increases upon immune stimulation and then rapidly decreases.

5. A method for treating a cancer patient, comprising the following steps (1) and (2) :
step (1) of evaluating whether the intracellular calcium ion concentration transiently increases or continuously increases when mononuclear cells (PBMC) contained in peripheral blood obtained from a cancer patient are subjected to immune stimulation;
step (2) of assuming that the combined treatment method using at least one member selected from the group consisting of phenformin, buformin and metformin, and one or more of other cancer treating agents is highly likely to exert a therapeutic effect when the intracellular calcium ion concentration of PMBC transiently increases upon immune stimulation and then rapidly decreases; and
step (3) of administering at least one member selected from the group consisting of phenformin, buformin and metformin, and one or more of other cancer treating agents to a patient who is assumed to have a high possibility of ensuring the therapeutic effect in step (2).

6. An agent for increasing intracellular calcium ion concentration in CD8T cells by immune stimulation, the agent comprising at least one member selected from the group consisting of phenformin, buformin and metformin.

7. The agent for increasing intracellular calcium ion concentration in CD8T cells by immune stimulation according to claim 6, wherein the agent selectively increases intracellular calcium ion concentration in CD8T cells in a tumor tissue.

8. An agent for selectively increasing the function of effector memory (EM) and effector (eff) in a tumor tissue, the agent comprising at least one member selected from the group consisting of phenformin, buformin and metformin.

9. A cancer treating agent to be administered to a cancer patient having a cancer tissue with a glucose concentration of 0.5 to 1.5 mM, the agent comprising at least one member selected from the group consisting of phenformin, buformin and metformin.

10. A method for monitoring effects of a cancer treating agent, comprising the following steps (i) to (iii):
step (i) of separating intratumor lymphocytes from a cancer tissue;
step (ii) of analyzing a glucose transporter (Glut1) expression level of the separated tumor-infiltrating lymphocytes (i); and
step (iii) of determining or evaluating whether a cancer treating agent that has already been administered to a cancer patient is effective based on the analyzed Glut1 expression level (as used herein, "a cancer tissue" means a cancer tissue removed from a cancer patient after administration of a cancer treating agent).

11. The method for monitoring effects of a cancer treating agent according to claim 9, wherein the cancer treating agent is selected from the group consisting of cancer vaccines, immune checkpoint inhibitors, phenformin, buformin and metformin.

12. The method for monitoring effects of a cancer treating agent according to claim 10 or 11, wherein the Glut1 expression level is analyzed by a flow cytometer.

13. The method for monitoring effects of a cancer treating agent according to any one of claims 10 to 12, wherein the tumor-infiltrating lymphocytes are CD8T cells.

14. The method for monitoring effects of a cancer treating agent according to any one of claims 10 to 13, wherein, in step 3, the glucose transporter (Glut1) expression level is analyzed after the separated tumor-infiltrating lymphocytes are cultured in a medium having a glucose concentration of 0.1 mM or more for at least two hours.
